(19) Europäisches Patentamt
European Patent Office
Office européen des brevets

(11) Publication number: 0 264 232 A1

# (12) EUROPEAN PATENT APPLICATION

(21) Application number: 87308943.7

(22) Date of filing: 09.10.87

(51) Int. Cl.[4]: C07D 205/08 , A61K 31/395

(30) Priority: 17.10.86 JP 246639/86

(43) Date of publication of application: 20.04.88 Bulletin 88/16

(84) Designated Contracting States: AT BE CH DE FR GB IT LI LU NL SE

(71) Applicant: TAISHO PHARMACEUTICAL CO. LTD
24-1 Takata 3-chome Toshima-ku
Tokyo 171(JP)

(72) Inventor: Kawashima, Yutaka
17431-1, Akoda
Tatebayashi-shi(JP)
Inventor: Satoh, Masakazu
Ekimae Puraza 6-205 15-1, Akamidai-2-chome
Konosu-shi(JP)
Inventor: Hatada, Yuichi
8-17, Minamimagome-4-chome
Ota-ku Tokyo(JP)
Inventor: Hazato, Fumiko
Kopo Sanraizu 203 41-7, Haraichi
Ageo-shi(JP)
Inventor: Nakashima, Yoshimoto
18-16, Gobancho
Ageo-shi(JP)
Inventor: Sota, Kaoru
1158-11, Shimotomi
Tokorozawa-shi(JP)

(74) Representative: Ellis, Edward Lovell et al
MEWBURN ELLIS & CO. 2/3 Cursitor Street
London EC4A 1BQ(GB)

## (54) 2-Azetidinone derivatives.

(57) 2-Azetidinone derivatives represented by the general formula

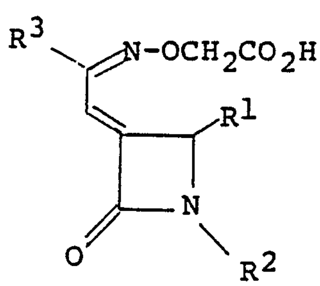

wherein $R^1$ is a lower alkyl group or an optionally substituted phenyl group, and $R^2$ is a lower alkyl group, a cycloalkyl group, an optionally substituted phenyl group, an optionally substituted benzyl group or a 1-

EP 0 264 232 A1

phenethyl group, and $R^3$ is a lower alkyl group, are useful as blood platelet aggregation inhibiting agents.

# 2-AZETIDINONE DERIVATIVES

## BACKGROUND OF THE INVENTION

### 1. FIELD OF THE INVENTION

The present invention relates to 2-azetidinone derivatives having blood platelet aggregation inhibiting activity.

### 2. Description of the Prior Art

Although some compounds having azetidinone skeleton which show antibacterial activity have been known in the past, any azetidinone derivative showing blood platelet aggregation inhibiting activity has not been yet reported.

## SUMMARY OF THE INVENTION

As a result of earnest researches to blood platelet aggregation inhibiting activity of the compounds having an azetidinone skeleton, the present inventors have found novel 2-azetidinone derivatives having blood platelet aggregation inhibiting activity, and the present invention has been completed.

An object of the present invention is to provide 2-azetidinone derivatives represented by the general formula

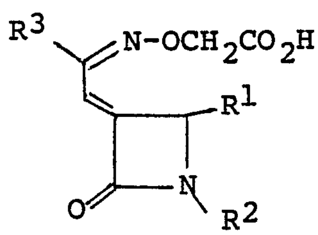

I

wherein $R^1$ is a lower alkyl group or a group of the formula

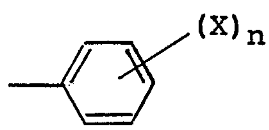

(wherein X is a hydrogen atom, a halogen atom, a lower alkyl group, a lower alkoxy group or a cyano group, and n is 1 or 2), and $R^2$ is a lower alkyl group, a cycloalkyl group, a group of the formula

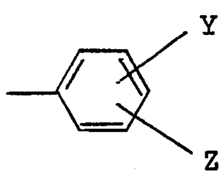

(wherein Y and Z are the same or different, and each is a hydrogen atom, a halogen atom, a lower alkyl group, a lower alkoxy group, an alkoxycarbonyl group or a dichloroacetyl group), a benzyl group, a benzyl group substituted by a lower alkyl group, a lower alkoxy group or halogen atom on the ring, or a 1-phenethyl group, and $R^3$ is a lower alkyl group.

Other object of the present invention is to provide blood platelet aggregation inhibiting agents containing the compound of formula I.

DETAILED DESCRIPTION OF THE INVENTION

In the present invention, the term "lower alkyl group" refers to those having 1 to 4 carbon atoms such as, for example, a methyl group, an ethyl group, a propyl group, an isopropyl group, a butyl group, an isobutyl group and the like. The term "lower alkoxy group" refers to those having 1 to 3 carbon atoms such as, for example, a methoxy group, an ethoxy group, a propoxy group and the like. The term "halogen atom" refers to a fluorine atom, a chlorine atom, a bromine atom and an iodine atom.

Preferred compounds of formula I are those wherein $R^1$ is a methyl group, a phenyl group or a 2-methoxyphenyl group, $R^2$ is a phenyl group, a 2,6-dimethylphenyl group or a 2-methyl-5-chlorophenyl group, and $R^3$ is a methyl group.

The compounds of the present invention can be easily prepared, for example, as follows: at first, a 2-azetidinone derivative of the general formula

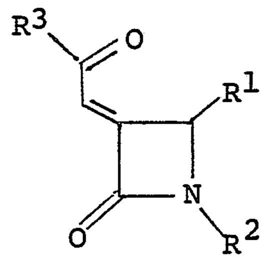

II

wherein $R^1$, $R^2$ and $R^3$ are as defined above, is reacted with benzhydryl 2-aminooxyacetate [$H_2NOCH_2CO_2CH(C_6H_5)_2$] in a reaction-inert solvent in the presence of a catalyst to give the oxime derivative represented by the general formula

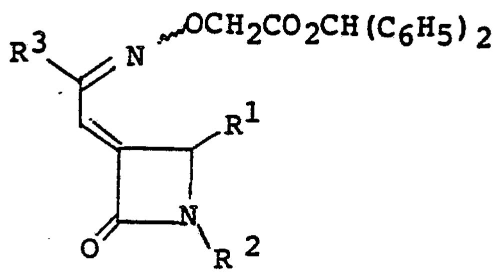

III

wherein $R^1$, $R^2$ and $R^3$ are as defined above. Examples of the reaction-inert solvent are alcohols (e.g., methanol, ethanol and the like), tetrahydrofuran, chloroform, methylene chloride, benzene and the like. Examples of the catalyst are amine salts (e.g., triethylamine hydrochloride, pyridine hydrochloride and the like), acetic acid, magnesium sulfate and the like. The reaction temperature is from 0°C to the reflux temperature of the solvent, preferably from room temperature to the reflux temperature of the solvent.

Next, the oxime derivative of formula III is treated with an acid such as trifluoroacetic acid, hydrochloric acid and the like for elimination of benzhydryl ester to give the compound of formula I of the present invention.

The configuration of the iminoalkylidene substituent of especially useful compounds of the present invention is E-form, and the configuration due to the asymmetric carbon atom at the 4-configuration is dl-form.

The compound of formula II can be easily prepared, for example, by a reaction (i.e., Wittig Reaction) of a compound of the general formula

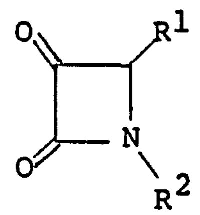

IV

wherein $R^1$ and $R^2$ are as defined above, with a Wittig reagent represented by the formula

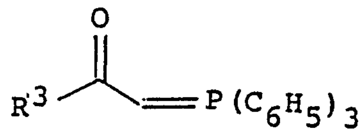

V

wherein $R^3$ is as defined above.

Reaction solvents used in this reaction are those used in the ordinary Wittig Reaction such as, for example, benzene, ethyl ether, tetrahydrofuran, toluene, chloroform, methylene chloride, dimethoxyethane and the like. The reaction temperature is from -30°C to the temperature of the boiling point of the solvent used, preferably from 0°C to the reflux temperature of the solvent. The reaction time depends on the starting material, the Wittig reagent or the reaction temperature, but usually it is from 2 to 48 hours, and the reaction may be stopped after the disappearance of the starting material observed by using thin layer silica gel column chromatography.

Some of the compounds of formula IV are known, and some are new and can be prepared by the methods described in the literature [e.g., Tetrahedron Letters, Vol. 25 (No. 42), page 4733 (1984)].

It is recognized that the compounds of the present invention have excellent blood platelet aggregation inhibiting activity and very poor bleeding tendency as side-effect, and therefore, they are useful as blood platelet aggregation inhibiting agents. For the purpose, these compounds can be administered orally or parenterally in a conventional dosage form such as tablets, powders, granules, capsules, solutions, emulsions, suspensions, injectional solutions and the like, each of which can be prepared by conventional pharmaceutical practices.

The dosage used as blood platelet aggregation inhibiting agents to human depends on the age, weight or response of patient, administration route or time of administration, but usually it may be from 10 to 3000 mg per day.

The $LD_{50}$ of the compound of formula I in mouse is more than 5000 mg/kg.

Next, following experiments illustrate concretely excellent blood platelet aggregation inhibiting activity and the prolonging effect of bleeding time of the compound of the present invention.

Experiment 1 [ex vivo test in rat]

Male Wister strain rats were administered orally with a suspension of the test drug in 0.2% carboxymethylcellulose. After 3 hours, the abdominal cavity exposed under pentobarbital anesthisia (40 mg/kg, i.p.), and nine volumes of blood mixed with one volume of 3.2% sodium citrate were collected from ventral artery. The blood was centrifuged at 150 g at room temperature for 15 minutes to give platelet rich plasma (PRP), and the remaining blood was centrifuged at 1500 g for 10 minutes to give platelet poor plasma (PPP) as a supernatant.

To 275 µl of PRP was added 25 µl of the aggregation-inducing substance [adenosine diphosphate (ADP) : final concentration 5 µM, or collagen : final concentration 5 µg/ml] at 37°C with stirring of 1000 rpm, and the maximum aggregation rate was measured for 5 minutes by means of the blood platelet aggregation ability measurement apparatus (Aggricoda TM•PA-3210, Kyoto Dai-ichi Kagaku), and the inhibition rate was calculated to the maximum aggregation rate of the rat which was administered with 0.2% carboxymethylcellulose as a control. The results were expressed as compared with 100 of the inhibition rate of the same amount of ticlopidine (positive control) as that of the test drug.

The compound numbers in Table 1 correspond to those in the Examples described below.

Table 1

| Compound No. | ADP | Collagen |
| --- | --- | --- |
| 2 | 119.8 | 94.5 |
| 5 | 118.5 | 47.6 |
| 12 | — | 123.6 |
| 13 | 65.5 | 137.5 |
| ticlopidine | 100 | 100 |

Experiment 2 [prolonging test of the bleeding time in mouse]

Six male ICR strain mice weighing 20 g for each group were administered orally with 300 mg/kg of the test drug (all the test drugs were used in the form of the suspension in 0.5% CMC). Two hours after administration, 5 mm of the tail from the top was cut under pentobarbital anesthesia, and the bleeding was observed by tapping at the cutting site with a filter paper every 15 seconds. The time when the bleeding was observed stopping for one minute is defined as the arrest point of bleeding, and the duration required from the time when the cutting was done to the arrest point of bleeding is defined as the bleeding time. The observation was carried out up to 1200 seconds. Ticlopidine was used as a positive control.

The results were shown in Table 2. The compound number in Table 2 corresponds to that in the Examples described below.

Table 2

| Compound No. | Bleeding time ± standard error |
| --- | --- |
| 1 | 417.5 ± 171.22 |
| ticlopidine | 1127.5 ± 72.50 (note) |
| the solvent | 305.0 ± 77.23 |

(Note) $P < 0.05$ by Mann and Whitney's U test.

The following Examples illustrate the method for preparing the compound of the present invention in more detail.

Example 1

To a solution of 2.35 g of acetylmethylene triphenylphosphorane in 200 ml of benzene was added at room temperature under a nitrogen atmosphere a solution of 1.75 g of 1,4-diphenyl-2,3-azetidinedione in 40 ml of benzene, and the mixture was stirred overnight. After completion of the reaction, the benzene was evaporated, and the residue was applied to silica gel column chromatography (eluent; methylene chloride). The desired fractions were combined, the solvent was evaporated, and the residue was recrystallized from ethanol to give 1.33 g of (E)-3-(2-oxopropylidene)-1,4-diphenyl-2-azetidinone as pale yellow needles.

m.p. 157.5 - 158.5°C

To 15 ml of ethanol were added 1.23 g of (E)-3-(2-oxopropylidene)-1,4-diphenyl-2-azetidinone, 1.13 g (1 equivalent) of benzhydryl 2-aminooxyacetate and 630 mg (1.1 equivalents) of triethylamine hydrochloride, and the mixture was heated at reflux for 2 hours. The solvent was evaporated under reduced pressure, the residue was extracted with methylene chloride, and extract was washed with, in turn, dilute hydrochloric acid, a saturated aqueous sodium bicarbonate solution and a saturated aqueous sodium chloride solution. After drying over anhydrous magnesium sulfate, the solvent was evaporated under reduced pressure to give 2.1 g of 1,4-diphenyl-3-(2-diphenylmethoxycarbonylmethoxyimino)propylidene-2-azetidinone, which is a mixture of anti-and syn-forms.

In 3 ml of anisole was dissolved 2.1 g of 1,4-diphenyl-3-(2-diphenylmethoxycarbonylmethoxyimino)-propylidene-2-azetidinone obtained above. To the cooled (0°C) solution was added dropwise 10 ml of trifluoroacetic acid, the mixture was stirred at the same temperature for an hour, and then the trifluoroacetic acid was evaporated without heating. The residue was extracted with methylene chloride, and the extract was dried over anhydrous magnesium sulfate. The solvent was evaporated, and the residue was applied to an activated charcoal column (eluted first with methylene chloride then with 20% methanol/methylene chloride) to give 1.08 g of 3-(2-carboxymethoxyiminopropylidene)-1,4-diphenyl-2-azetidinone.

This compound was washed with diethyl ether and recrystallized from ethanol to give of 480 mg of the anti-form of 3-(2-carboxymethoxyiminopropylidene)-1,4-diphenyl-2-azetidinone (as pale yellow plisms, Compound 1).

m.p. 189 - 190°C

In a similar procedure, the compounds listed in Table 3 were obtained.

Table 3

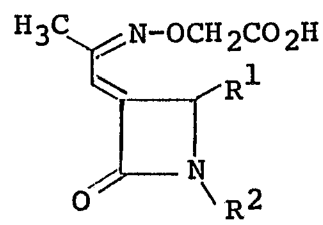

| Compound No. | $R^1$ | $R^2$ | m.p. (°C) |
|---|---|---|---|
| 2 | methyl | phenyl | 148.5-149.5 |
| 3 | methyl | o-methoxyphenyl | >250 |
| 4 | phenyl | o-methylphenyl | 137-138 |
| 5 | phenyl | 2,6-dimethylphenyl | 193-194 |
| 6 | phenyl | o-chlorophenyl | 140-142 |
| 7 | phenyl | 2-methyl-5-chlorophenyl | 152.5-154.5 |
| 8 | phenyl | p-methoxyphenyl | 186-187 |
| 9 | phenyl | 2-phenethyl | oily |
| 10 | phenyl | o-chloropbenzyl | oily |
| 11 | p-bromophenyl | p-methylphenyl | 172-174 |

- Cont'd -

Table 3 (Cont'd)

| | | | |
|---|---|---|---|
| 12 | m-methoxyphenyl | phenyl | 168-169 |
| 13 | o-methoxyphenyl | o-methylphenyl | 189-191 |
| 14 | p-cyanophenyl | o-methylphenyl | oily |
| 15 | p-methylphenyl | phenyl | 192.5-193 |
| 16 | 3,4-dichlorophenyl | phenyl | 162.5-167.5 |
| 17 | phenyl | p-fluorophenyl | 208-209 |
| 18 | 3,4-dimethoxyphenyl | phenyl | 186.5-188.5 |
| 19 | 2,6-dichlorophenyl | phenyl | 189.5-190.5 |
| 20 | phenyl | o-fluoropphenyl | 163-166.5 |
| 21 | phenyl | o-methoxyphenyl | 100-103 |
| 22 | phenyl | o-methoxycarbonylphenyl | 193-195 |
| 23 | phenyl | 3-methyl-4-chlorophenyl | 193-195 |
| 24 | phenyl | p-dichloroacetylphenyl | 196-197 |
| 25 | phenyl | o-fluorobenzyl | 114-116 |
| 26 | phenyl | o-methoxybenzyl | 50-51 |

- Cont'd -

Table 3 (Cont'd)

| | | | |
|---|---|---|---|
| 27 | phenyl | p-methylbenzyl | oily |
| 28 | phenyl | 2,4-dichlorobenzyl | oily |
| 29 | phenyl | cyclohexyl | 70-76 |
| 30 | methyl | isobutyl | oily |

$^1$H-NMR values of the oily substances descibed above are as follows:

| Compound No. | $^1H$-NMR ($CDCl_3$) δ(ppm) |
|---|---|
| 9 | 1.15 and 1.77 (3H, d, J=7Hz), 1.60 and 1.64 (3H, s), 4.30 and 5.16 (1H, m), 4.58 (2H, bs), 4.91 and 4.96 (1H, d, J=2Hz), 6.56 and 6.60 (1H, d, J=2Hz), 7.1-7.4 (10H, m) |
| 10 | 1.67 (3H, s), 4.21 (1H, d, J=14Hz), 4.53 (1H, d, J=6Hz), 4.57 (1H, d, J=6Hz), 4.79 (1H, d, J=14Hz), 5.25 (1H, d, J=2Hz), 6.57 (1H, d, J=2Hz), 7.2-7.4 (9H, m) |
| 14 | 1.86 (3H, s), 2.34 (3H, s), 4.61 (2H, s), 5.80 (1H, d, J=1Hz), 6.10 (1H, bs), 6.71 (1H, d, J=1Hz), 7.05-7.15 (4H, m), 7.50 (2H, d, J=8Hz), 7.63 (2H, d, J=8Hz) |
| 27 | 1.66 (3H, s), 2.35 (3H, s), 3.71 (1H, d, J=15Hz), 4.3 (1H, bs), 4.39 (2H, s), 4.86 (1H, d, J=15Hz), 5.01 (1H, d, J=2Hz), 6.60 (1H, d, J=2Hz), 7.04 (2H, d, J=9Hz), 7.14 (2H, d, J=9Hz), 7.2-7.4 (5H, m) |
| 28 | 1.58 (3H, s), 3.7 (1H, bs), 4.13 (1H, d, J=15Hz), 4.48 (2H, s), 4.66 (1H, d, J=15Hz), 5.39 (1H, d, J=2Hz), 6.43 (1H, d, J=2Hz), 7.2-7.4 (7H, m), 7.53 (1H, d, J=2Hz) |
| 30 | 0.93 (3H, d, J=6Hz), 0.97 (3H, d, J=6Hz), |

1.42 (3H, d, J=6Hz), 1.93 (1H, m), 2.05 (3H, s), 2.94 (1H, dd, J=14Hz, 8Hz), 3.32 (1H, dd, J=14Hz, 8Hz), 4.39 (1H, m), 4.73 (2H, s), 6.40 (1H, d, J=1Hz), 6.74 (1H, bs)

Example 2

Following the similar procedure to that of Example 1 using (E)-3-(2-oxobutylidene)-1,4-diphenyl-2-azetidinone and (E)-3-(2-oxobutylidene)-1-benzyl-4-phenyl-2-azetidinone, respectively, in place of (E)-3-(2-oxopropylidene)-1,4-diphenyl-2-azetidinone, the following compounds were obtained.

3-(2-carboxymethoxyiminobutylidene)-1,4-diphenyl-2-azetidinone, m.p. 88 - 92°C.

3-(2-carboxymethoxyiminobutylidene)-1-benzyl-4-phenyl-2-azetidinone, as an oil.

$^1$H-NMR ($CDCl_3$) δ(ppm)

0.73 (3H, t, J = 7.5Hz), 2.17 (2H, q, J = 7.5Hz), 3.80 (1H, d, J = 15Hz), 4.50 (2H, S), 4.85 (1H, d, J = 15Hz), 5.00 (1H, d, J = 2Hz), 6.52 (1H, d, J = 2Hz), 7.1-7.5 (10H, m), 10.5 (1H, brs).

## Claims

1. 2-Azetidinone derivatives represented by the general formula

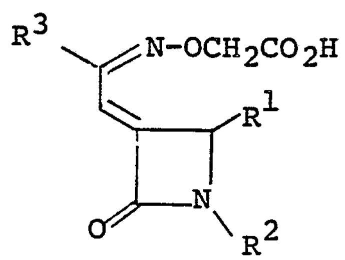

wherein $R^1$ is a lower alkyl group or a group of the formula

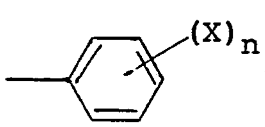

(wherein X is a hydrogen atom, a halogen atom, a lower alkyl group, a lower alkoxy group or a cyano group, and n is 1 or 2), and $R^2$ is a lower alkyl group, a cycloalkyl group, a group of the formula

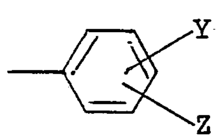

(wherein Y and Z are the same of different, and each is a hydrogen atom, a halogen atom, a lower alkyl group, a lower alkoxy group, an alkoxycarbonyl group or a dichloroacetyl group), a benzyl group, a benzyl group substituted by a lower alkyl group, a lower alkoxy group or halogen atom on the ring, or a 1-phenethyl group, and $R^3$ is a lower alkyl group.

2. Blood platelet aggregation inhibiting agents containing 2-azetidinone derivatives represented by the general formula

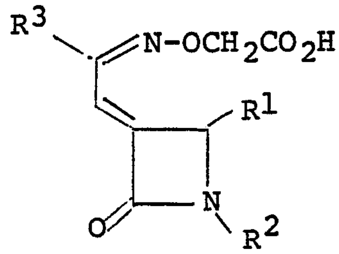

wherein $R^1$ is a lower alkyl group or a group of the formula

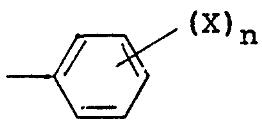

(wherein X is a hydrogen atom, a halogen atom, a lower alkyl group, a lower alkoxy group or a cyano group, and n is 1 or 2), and $R^2$ is a lower alkyl group, a cycloalkyl group, a group of the formula

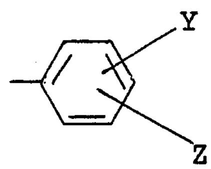

(wherein Y and Z are the same or different, and each is a hydrogen atom, a halogen atom, a lower alkyl group, a lower alkoxy group, an alkoxycarbonyl group or a dichloroacetyl group), a benzyl group, a benzyl group substituted by a lower alkyl group, a lower alkoxy group or halogen atom on the ring, or a 1-phenethyl group, and $R^2$ is a lower alkyl group.

3. A 2-azetidinone derivative according to Claim 1, wherein the iminoalkylidene substituent has the E-configuration.

4. A 2-azetidinone derivative according to Claim 1 or Claim 3, wherein the configuration due to the asymmetric carbon atom at the 4-point is of the dl-form.

5. A process for producing a 2-azetidinone derivative of the formula given and defined in Claim 1, which comprises

a) reacting a 2-azetidinone derivative of the formula

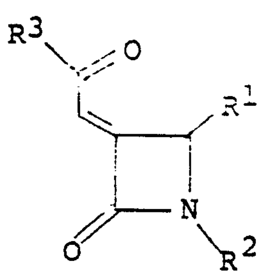

II

wherein $R^1$, $R^2$ and $R^3$ are as defined in Claim 1, with benzhydryl 2-aminooxyacetate to give an oxime derivative of the formula

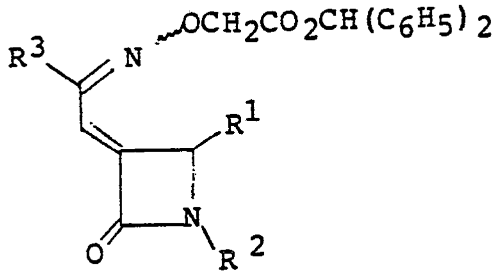

III

wherein $R^1$, $R^2$, and $R^3$ are as defined in Claim 1, and

b) reacting the oxime derivative of the formula (III) with an acid.

6. A process according to Claim 5, wherein the reaction step a) is carried out in the presence of a catalyst.

7. A process according to Claim 5 or Claim 6 which includes the preliminary step of reacting a compound of the formula

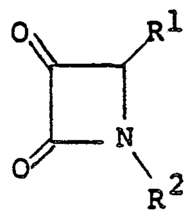

wherein $R^1$ and $R^2$ are as defined in Claim 1, with a Wittig reagent of the formula

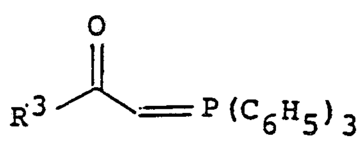

wherein $R^3$ is as defined in Claim 1, to obtain the said 2-azetidinone derivative of the formula II.

8. A 2-azetidinone derivative of the formula given and defined in Claim 1 for use in a pharmaceutical.

9. A 2-azetidinone derivative of the formula given and defined in Claim 1 for use as a blood platelet aggregation inhibiting agent.

10. A pharmaceutical composition comprising a 2-azetidinone of the formula given and defined in Claim 1 and a pharmaceutically acceptable diluent or carrier.

European Patent Office

# EUROPEAN SEARCH REPORT

Application number

EP 87308943.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | TETRAHEDRON, vol. 41, no. 2, 1985<br>MORI et al. "New synthesis of β-lactames"<br>pages 375-385<br>* Pages 381, 385 (compounds 20a, 20b, 20c, 20c') *<br>-- | 1,3,4,7 | C 07 D 205/08<br>A 61 K 31/395 |
| A | LIEBIGS ANNALEN DER CHEMIE, Heft 5, 1983<br>H.-H. OTTO et al.: "Darstellung und Stereochemie von 3-(α-Hydroxybenzyl)-1,4-diphenyl-2-azetidinonen"<br>pages 1152-1168<br>* Pages 1153, 1158 (compounds 3,5); pages 1165-1168 (compounds 4,4f,8) *<br>-- | 1,3,4,7 | |
| A | ARCHIV DER PHARMAZIE, vol. 319, no. 3, March 1986<br>BERGMANN et al.: "Zur N- unc C-Silylierung von β-Lactamen"<br>pages 203-216<br>* Pages 208, 214, 215 (compounds 14,15) *<br>-- | 1,3,4,7 | TECHNICAL FIELDS SEARCHED (Int. Cl.4)<br>C 07 D 205/00<br>A 61 K 31/00 |
| A | EP - A1 - 0 149 419 (NIPPON ZOKI PHARM.)<br>* Page 1, last two lines; page 2; claims 15-19 *<br>-- | 2,8-10 | |

The present search report has been drawn up for all claims

| Place of search | Date of completion of the search | Examiner |
|---|---|---|
| VIENNA | 08-01-1988 | JANISCH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

# EUROPEAN SEARCH REPORT

Application number

EP 87308943.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | TETRAHEDRON LETTERS, vol. 25, no. 42, 1984<br>MANHAS et al. "A convenient synthesis of azetidine-2,3-diones"<br>pages 4733-6<br>* Page 4735 *<br>-- | 7 | |
| A | EP - A1 - 0 135 194 (TAKEDA)<br>* Claims 9,24 *<br>---- | 1,8,10 | |
| | | | TECHNICAL FIELDS SEARCHED (Int. Cl.4) |

The present search report has been drawn up for all claims

| Place of search | Date of completion of the search | Examiner |
|---|---|---|
| VIENNA | 08-01-1988 | JANISCH |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document